# EUROPEAN PATENT APPLICATION

(11) **EP 3 103 377 A1**
(43) Date of publication of application: **14.12.2016**
(21) Application number: 13895915.0
(22) Date of filing: 21.10.2013
(51) Int. Cl.: A61B 1/015

(54) **ENDOSCOPE WITH CONTINUOUS LUMINAL PERFUSION AND REFLUX FUNCTIONS**

(71) Applicant: Youcare Technology Co., Ltd. (WUHAN), East Lake Development Zone Wuhan Hubei 430223 (CN)
(72) Inventor: LONG, Gang, Wuhan Hubei 430223 (CN); HU, Xuecheng, Wuhan Hubei 430223 (CN); LI, Ying, Wuhan Hubei 430223 (CN); WANG, Shaogang, Wuhan, Hubei 430223 (CN); YU, Xiao, Wuhan, Hubei 430223 (CN)
(74) Representative: Behr, Wolfgang
(86) International application number: PCT/CN2013/085544
(87) International publication number: WO 2015/058329

(57) **Abstract**

This invention provides an endoscope with continuous luminal perfusion and reflux functions which comprises an endoscope handle (1), a hard sheath and a soft sheath (6). The proximal end of the hard sheath is connected with the endoscope handle (1). The soft sheath (6) is located within the hard sheath and the soft sheath (6) can axially extend from the inside of the hard sheath and rotate circumferentially. The endoscope is characterized in that a drainage channel (9) is axially arranged between the outer wall of the soft sheath (6) and the inner wall of the hard sheath. A water inlet (8) communicated with the drainage channel (9) is circumferentially arranged on the distal end of the hard sheath, and a water outlet (7) communicated with the drainage channel (9) is arranged on the rear-middle part of the hard sheath. The endoscope is simple in structure and convenient to process and assemble, and the adoption of the technical solution that the drainage channel (9) is arranged between the outer wall of the soft sheath (6) and the inner wall of the hard sheath enables the endoscope to utilize the drainage channel (9) for drainage and perfusion during an operation.

## Description

### TECHNICAL FIELD

The invention relates to the field of medical instrument, and more particularly to an endoscope with continuous luminal perfusion and reflux functions.

### BACKGROUND OF THE INVENTION

In the field of clinical medicine, one of most commonly used diagnosis and treatment methods is conducting treatment with an endoscope, which is called minimally invasive treatment due to the small damage to patient. During the medical treatment with the endoscope, in order to have a clear operation field, physiological saline is injected to human organs under a suitable pressure. In the traditional endoscope, a water discharge channel is omitted, or the water discharging and the water feeding share the same channel, so that a circulation state can not be formed. When facing a complex case, with the increasing operation time, the retention time of the endoscope in the body of the patient is increased and the amount of the physiological saline injected into the body of the patient is also increased correspondingly. If the amount of the physiological saline injected into the body of the patient is too much, it may cause a surgical infection, even the expanding of the organs, which results in a damage to the organs of the patient. Thus, the traditional endoscope has a high requirement on the technology and experiences of physician.

In addition, during the minimally invasive treatment, the size of the cavity channel formed by the surgical instruments when entering into the body of the patient will greatly affect the postoperative recovery of the patient body, so that on the premise of meeting the requirements of the operation, the outer diameter of the endoscope is smaller, the postoperative recovery is more beneficial. Therefore, it is necessary to add the continuous luminal perfusion and reflux functions to the endoscope.

### SUMMARY OF THE INVENTION

The object of the present invention is to provide an endoscope with continuous luminal perfusion and reflux functions to overcome the defects mentioned in the background. The endoscope has the advantages of simple operation and high operation safety.

To achieve the above object, the present invention provides an endoscope with continuous luminal perfusion and reflux functions. The endoscope comprises an endoscope handle, a hard sheath and a soft sheath, an inside end of the hard sheath is connected with the endoscope handle, the soft sheath is located within the hard sheath, the soft sheath can axially extend from the inside of the hard sheath and rotate circumferentially, a drainage channel is axially arranged between an outer wall of the soft sheath and an inner wall of the hard sheath, a water inlet communicated with the drainage channel is circumferentially arranged on an outer end part of the hard sheath, a water outlet communicated with the drainage channel is arranged on a rear-middle part of the hard sheath.

Preferably, a head end cap 5 is arranged on an outer end part of the soft sheath, the head end cap 5 can be blocked with an end part of the hard sheath, and the head end cap 5 is thicker than the soft sheath.

Preferably, the outer diameter of the head end cap 5 is equal to the outer diameter of the hard sheath to which the head end cap 5 corresponds.

Preferably, a gap is arranged between the outer wall of the soft sheath and the inner wall of the hard sheath, the drainage channel is located in the gap.

Preferably, the water inlet is arranged on the side wall of the outer end of the hard sheath.

Preferably, the water inlet is consisted of small holes which are uniformly distributed on the side wall of the outer end of the hard sheath.

Preferably, the hard sheath is consisted of a thin sheath 4 and a thick sheath 2, the thin sheath 4 and the thick sheath 2 are coaxially integrated, the outer diameter and the inner diameter of the thick sheath 2 are respectively greater than the outer diameter and the inner diameter of the thin sheath 4, a sheath ring 3 is sleeved on a binding portion between the thin sheath 4 and the thick sheath 2, the sheath ring 3 is sleeved on the thin sheath 4 and the thick sheath 2, the inner surface of the sheath ring 3 which corresponds to the thick sheath is in a shape of a cone with a larger outer portion and a small inner portion, the water outlet is arranged on a sheath wall of the thick sheath which locates in the conical area.

Preferably, the appearance of the sheath ring 3 is in a shape of an inward concave arc handle.

The invention has the following advantages:

The endoscope of this invention is simple in structure and convenient to process and assemble. Especially, it adopts a technical solution that the discharging channel is provided between the outer wall of the soft sheath and the inner wall of the hard sheath, so that the drainage channel can be used for drainage and perfusion during the operation. The water is injected into the organs via a cavity channel of the soft sheath. The water in the organs enters into the small hole on the sheath wall of the head end of the hard sheath under a negative pressure. The water can be discharged from the gap betwee n the hard sheath and the soft sheath, and thus a continuous reflux is formed. When the endoscope with continuous luminal perfusion and reflux functions is inserted into the human body during the operation, the physiological saline in the organs flows into the drainage channel via the water inlet arranged on the external end of the hard sheath and is discharged from the water outlet located on the middle part of the hard sheath. As the water outlet faces on the direction of water discharging and is blocked with the sheath ring 3, it can prevent the sewage which overflows from the water outlet from spouting and polluting the environment.

In addition, the outer diameter of the head end cap 5 is equal to the outer diameter of the hard sheath to which the head end cap corresponds, so that the endoscope can enters into the body smoothly and will not damage the human tissues. The sheath ring is in the shape of inner concave arc. One end of the sheath ring is sleeved on the thin sheath and the other end thereof is sleeved on the thick sheath, so that it facilitates the pulling and prettifies the joints.

### BRIEF DESCRIPTION OF THE DRAWINGS

FIG. 1 is a front view according to the present invention;
FIG. 2 is an axial sectional view of FIG.1;
FIG. 3 is an axial sectional view of FIG.1 along line C-C;
FIG.4 is an enlarged view of part B shown in FIG.2;

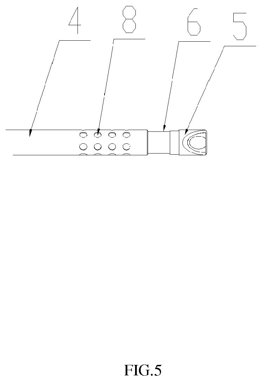

FIG.5 is an enlarged view of part A shown in FIG.1.

In the figures, 1. endoscope handle, 2. thick sheath, 3.sheath ring, 4. thin sheath, 5. head end cap, 6. soft sheath, 7. water outlet, 8. water inlet, 9. drainage channel.

### DETAILED DESCRIPTION OF THE EMBODIMENTS

The invention is further illustrated in more detail in the light of the drawings and embodiments, which are not intended to define the protection scope of the invention.

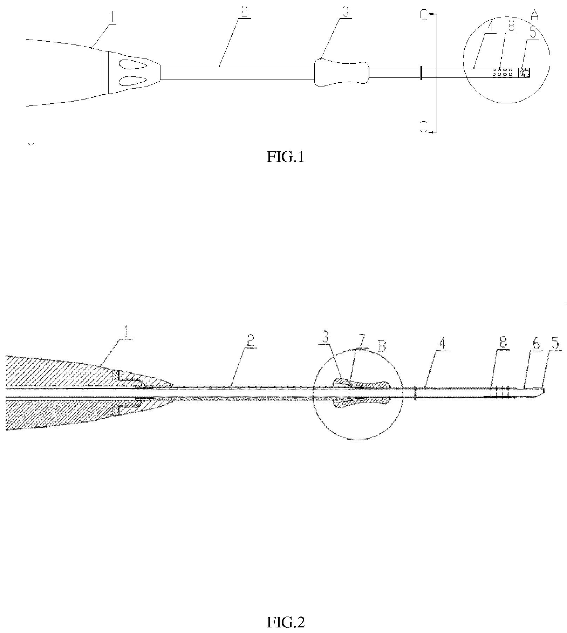

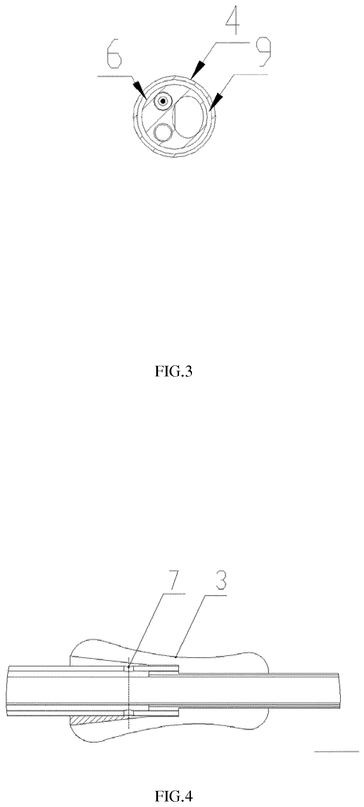

As shown from FIGS.1-4, this embodiment relates to an endoscope with continuous luminal perfusion and reflux functions. The endoscope comprises a head end cap 5, a thin sheath 4, a thick sheath 2, a soft sheath 6, a sheath ring 3 and an endoscope handle 1. A hard sheath is consisted of the thin sheath 4 and the thick sheath 2. The material of the hard sheath is the existing medical material or other new materials. The thin sheath 4 and the thick sheath 2 are coaxially integrated. The outer diameter and the inner diameter of the thick sheath 2 are respectively greater than the outer diameter and the inner diameter of the thin sheath 4. A part of the root of the thin sheath 4 is inserted into the inner hole of the thick sheath 2. The sheath ring 3 is sleeved on the binding portion between the thin sheath 4 and the thick sheath 2. The appearance of the sheath ring 3 is in a shape of an inward concave arc handle. One end of the inner surface of the sheath ring 3 is in an arc surface and the other end thereof is a conical surface. The arc surface of the sheath ring 3 is partially sleeved on the thin sheath 4 and the other part of the conical surface is located outside of the thick sheath 2. The inner surface of the sheath ring 3 which corresponds to the thick sheath 2 is in a shape of a cone with a larger outer part and a small inner part. A water outlet 7 is arranged on the sheath wall of the thick sheath which locates in the conical area. The number and the size of the water outlet are not limited. Moreover, a water valve can be mounted on the water outlet to adjust the amount of the drainage. In this embodiment, the water outlet is preferably consisted of small holes which are uniformly distributed on the outer side wall of the hard sheath. The other end of the thick sheath 2 is connected with the endoscope handle 1. The structures of the endoscope handle 1, the inside of the endoscope handle and other parts of the endoscope are same with that disclosed in the international applicant No. PCT/CN2010/076974, with a title "Combined Flexible and Rigid Endoscope".

In this embodiment, the soft sheath 6 is inserted in to the thin sheath 4 and the thick sheath 2. The soft sheath 6 is made of medical polymers and it can extend from the outer end part of the thin sheath 4. The outer diameter of the soft sheath 6 is smaller than the inner diameter of the thin sheath 4. A gap with a width of 0.4mm is formed between them. The gap acts as the drainage channel 9. A water inlet 8 communicated with the drainage channel 9 is arranged on the sheath wall of the outer end part of the thin sheath 4. The water inlet is consisted of the small holes distributed on the outer side wall of the hard sheath. The diameter of the small hole is 1.0mm and the distance between a small hole and an adjacent small hole is 1.8mm.

In this embodiment, a head end cap 5 is arranged on an outer end part of the soft sheath. The head end cap 5 can be blocked with an end part of the thin sheath 4. The head end cap 5 is thicker than the soft sheath. The outer diameter of the head end cap 5 is equal to the outer diameter of thin sheath 4. When the two elements align, the surfaces of them are smooth, so that it is easy to enter into the human body. A through hole which corresponds to each channel of the soft sheath is arranged on the end surface of the head end cap 5. The head end cap 5 is sleeved on the soft sheath 6 and engaged with the soft sheath 6 tightly. The head end cap 5 is made of medical metal material, non-metallic material or other new materials.

During the operation, the endoscope of this invention enters into the human body. In order to maintain the clarity of the visual field in the operation, doctors will inject the physiological saline into the human body or organs via the endoscope. With the increased water injection, the pressure in the organs will be increased correspondingly. In such a case, a part of the water in the organs will enters into the gap between the soft sheath 6 and the thin sheath 4 via the water inlet 8 located on the front end of the thin sheath 4. The gap is equivalent to the drainage channel 9. Finally, the water is discharged out of the human body via the water outlet 7 located on the thick sheath 5. During the drainage, the sheath ring 3 located outside of the water outlet can prevent the sewage which overflows from the water outlet from spouting and polluting the environment. At the same time, the appearance of the sheath ring 3 is in a shape of an inward concave arc handle, and thus facilitates the holding of the doctors and the pulling of the soft sheath and the hard sheath.

Both the other components of the endoscope and the connection structure thereof are prior art, so that they are omitted here.

Several routine applications are listed as follows.

(1) The application on the urinary surgery: it can be applied on an ureteroscope, a percutaneous nephrolithotomy, a cystoscope and a laparoscope. The continuous luminal perfusion functions can reduce the pressure in kidney and maintain the clarity of the operation visual field to prevent the spread of malignant cells. It also can be applied on the surgery of the kidney stone, the renal cyst and renal tumor.

(2) The application on the neurosurgery: the soft sheath and the hard sheath are used in integrate and the continuous luminal perfusion functions can reduce the intracranial pressure to ensure the safety of the operation. It can be widely used in the treatment of hydrocephalus, the disease in ventricle, hypophysoma, aneurysm, intracranial hematoma and skull base, especially can be used to treat the separated hematoma under dura mater and can be used in the endoscopic percutaneous discography , and even can be used in the substantial tumor biopsy in brain, the cutting of small tumor, microvascular decompression for trigeminal neuralgia and neurotomy of vestibular nerve.

(3) The application on gynaecology: It can be used in an operation on fallopian tube with reproductive endoscope and hysteroscope. It also can be used in an optimization of the existing hysteroscope, laparoscope and colposcope. Furthermore, it can be used in the treatment of cervical lesions to understand the treatment effect and confirm that whether a new lesion arises or the lesion reappears. Moreover, it can be used in the check and treatment of abnormal uterine bleeding. It can be used in checking the myomectomy, polyps, endometrial cancer, abnormal ultrasound, infertility and family planning complication, physiological changes in endometrium caused by tamoxifen, previous IVF. It also can substitute hysteroscope to check the condition of endometrium, habitual abortion, uterine malformation, metrosynizesis,foreign matters in uterine cavity and ectopic pregnancy, cut the benign gynecologic tumor and treat the oophoritic cyst, the pelvic adhesion and ligation of oviduct.

(4) The application on the cordocentesis choledochoscope: it not only can be used in choledochoscope and can enters into the biliary tract or gallbladder to treat disease, but also can be used in treating the gallbladder polyps lesion and keep the function of gallbladder, so that it facilitates to improve the quality of life after the operation and reduce the complication after the operation.

In addition, it can be applied in forensic sursery, digestive system department, pancreatic surgery, vascular surgery, respiration department and orthopedics department etc.. Due to limitation of length, no more tautology here.

## Claims

1. An endoscope with continuous luminal perfusion and reflux functions, comprising an endoscope handle (1), a hard sheath and a soft sheath, an inside end of the hard sheath is connected with the endoscope handle (1), the soft sheath is located within the hard sheath, the soft sheath can axially extend from the inside of the hard sheath and rotate circumferentially, **characterized in that** a drainage channel is axially arranged between an outer wall of the soft sheath and an inner wall of the hard sheath, a water inlet communicated with the drainage channel is circumferentially arranged on an outer end part of the hard sheath, a water outlet communicated with the drainage channel is arranged on a rear-middle part of the hard sheath.

2. The endoscope with continuous luminal perfusion and reflux functions according to claim 1, **characterized in that** a head end cap (5) is arranged on an outer end part of the soft sheath, the head end cap (5) can be blocked with an end part of the hard sheath, the head end cap (5) is thicker than the soft sheath.

3. The endoscope with continuous luminal perfusion and reflux functions according to claim 2, **characterized in that** the outer diameter of the head end cap (5) is equal to the outer diameter of the hard sheath to which the head end cap corresponds.

4. The endoscope with continuous luminal perfusion and reflux functions according to claim 1, **characterized in that** a gap is arranged between the outer wall of the soft sheath and the inner wall of the hard sheath, the drainage channel is located in the gap.

5. The endoscope with continuous luminal perfusion and reflux functions according to any one of claims 1-4, **characterized in that** the water inlet is located on the side wall of the outer end of the hard sheath.

6. The endoscope with continuous luminal perfusion and reflux functions according to claim 5, **characterized in that** the water inlet is consisted of small holes which are uniformly distributed on the side wall of the outer end of the hard sheath.

7. The endoscope with continuous luminal perfusion and reflux functions according to any one of claims 1-4, **characterized in that** the hard sheath is consisted of a thin sheath (4) and a thick sheath (2), the thin sheath (4) and the thick sheath (2) are coaxially integrated, the outer diameter and the inner diameter of the thick sheath (2) are respectively greater than the outer diameter and the inner diameter of the thin sheath (4), a sheath ring (3) is sleeved on a binding portion between the thin sheath (4) and the thick sheath (2), the sheath ring (3) is sleeved on the thin sheath (4) and the thick sheath (2), the inner surface of the sheath ring (3) which corresponds to the thick sheath is in a shape of a cone with a larger outer portion and a small inner portion, the water outlet is arranged on a sheath wall of the thick sheath which locates in the conical area.

8. The endoscope with continuous luminal perfusion and reflux functions according to claim7, **characterized in that** the appearance of the sheath ring (3) is in a shape of an inward concave arc handle.
